# EUROPÄISCHE PATENTSCHRIFT

(11) **EP 2 619 366 B1**
(45) Veröffentlichungstag und Bekanntmachung des Hinweises auf die Patenterteilung: **26.11.2014**
(21) Anmeldenummer: 11758443.3
(22) Anmeldetag: 14.09.2011
(51) Int. Cl.: D21G 9/00, G01N 33/34, G01N 27/60

(54) **VERFAHREN ZUM REGELN DER FORMATION EINER FASERSTOFFBAHN**
METHOD FOR REGULATING THE FORMATION OF A FIBROUS WEB
PROCÉDÉ DE RÉGULATION DE LA FORMATION D'UNE BANDE DE MATIÈRE FIBREUSE

(30) Priorität: 20.09.2010 DE 102010041052
(43) Veröffentlichungstag der Anmeldung: 31.07.2013
(73) Patentinhaber: Voith Patent GmbH, 89522 Heidenheim (DE)
(72) Erfinder: BAUER, Armin, A-3107 St. Pölten (AT); KÄSER, Jürgen, 88364 Wolfegg (DE); WOHLMUTH, Alexander, 73614 Schorndorf (DE); HAAG, Jens, 89522 Heidenheim (DE); NAYDOWSKI, Christian, CH-4806 Wikon (CH); STAIGER, Martin, 89198 Westerstetten (DE); KRIECHBAUM, Günther, 89522 Heidenheim (DE); BRITZ, Herbert, 88276 Berg (DE)
(86) Internationale Anmeldenummer: PCT/EP2011/065940
(87) Internationale Veröffentlichungsnummer: WO 2012/038310

(56) Entgegenhaltungen:
- EP-A1- 1 801 288
- US-B1- 6 176 974

## Beschreibung

Die Erfindung betrifft ein Verfahren sowie eine Anlage zum Regeln der Formation einer Faserstoffbahn, bei der es sich insbesondere um eine Papier-, Karton- oder Tissuebahn handeln kann. Dabei wird die Formation der Faserstoffbahn als Regelgröße online gemessen und über eine automatische Formationsregelung auf einem vorgebbaren Sollniveau gehalten.

Als Formation bezeichnet man die Faserverteilung und Zusammensetzung in einer Faserstoffbahn. Die Formation wird allgemein auch als die "Durchsicht" z.B. des Papiers bezeichnet.

Die Prüfung und Beurteilung der Formation erfolgt in der Regel dadurch, dass das Papier durchleuchtet wird. Die Struktur und der Grad der Einheitlichkeit der Faserverteilung im Papier ist ein Maß für die Formation. Dabei wird unterschieden nach gleichmäßiger, ruhiger und klarer Durchsicht bei guten Papieren und ungleichmäßiger, wolkiger und sehr unruhiger Durchsicht bei weniger guten Papieren.

Die Formation einer Papier- oder Kartonbahn ist ein entscheidendes Qualitätsmerkmal, das die Weiterverarbeitung der Faserstoffbahn auf vielfältige Weise beeinflusst. Als Beispiel sei der Einfluss der Formation auf die Festigkeit und die Bedruckbarkeit des Endproduktes genannt.

Die Formation einer Papier- oder Kartonbahn wird zum einen durch den eingesetzten Rohstoff und zum Anderen, in nicht unerheblichem Umfang, durch die Blattbildung beeinflusst. Dabei ist die Blattbildung durch den Stoffauflauf und die Formiereinheit bestimmt. Die Formiereinheit kann beispielsweise ein Langsieb, einen Hybridformer oder einen modernen Doppelsiebformer umfassen.

Bei den Größen, die bei diesem Prozessschritt die Formation beeinflussen, kann es sich beispielsweise um die Stoffdichte, Vakua in der Formiereinheit, die Retention und die Wassermenge handeln, wie auch in der EP-A-1801288 bereits beschrieben.

Die Erfahrung zeigt, dass bereits geringfügige Änderungen in der Zusammensetzung und den Eigenschaften des verwendeten Faserstoffes erheblichen Einfluss auf die Formation haben können. Zudem erfordert jede Veränderung der Flächenmasse einer Papierbahn die Nachstellung der die Formation beeinflussenden Größen.

Aus den genannten Gründen ist die Formation ständigen Schwankungen unterworfen. Besonders ausgeprägt sind diese Formationsschwankungen bei Altpapier enthaltenden Produkten, was auf die entsprechenden Schwankungen der Rohstoffzusammensetzung zurückzuführen ist.

Spontan auftretenden Formationsänderungen wird heute hauptsächlich durch mechanisches Verstellen der Stoffauflauflippenöffnung begegnet. Hierdurch wird die Stoffkonsistenz im Zulauf zum Stoffauflauf verändert, sodass das Flächengewicht konstant gehalten werden kann.

Für die Verbesserung der Formation ist eine Vergrößerung der Lippenöffnung notwendig, was zu einem höheren Energieeinsatz führt, da das Durchsatzvolumen steigt. In der Folge sinkt aber die Gesamt- und Ascheretention nachteilig ab, wodurch mehr Retentionsmittel zugegeben werden müsste.

Betroffen sind ferner die Regelkreise für die automatische Dosierung von Retentionsmittel und der Wirkungsgrad der, das Siebwasser entstoffenden, nachfolgenden Aggregate.

Das Absinken der Retentionen macht die Zugabe erhöhter Mengen an Retentionsmittel erforderlich, wodurch auch die Formation durch stärkere Flockung beeinträchtigt wird. Durch diese sich selbst aufschaukelnden Regelkreise wird eine Prozessschwankung ausgelöst, die das gesamte wasserführende System der Papiermaschine (Stoffzentrale, Konstantteil, Prozesswasserrückgewinnung, Stoffaufbereitung) über mehrere Stunden destabilisiert.

Zur automatischen Regelung der Formation werden im Stand der Technik einige Online-Formationsmessungen und Regelung offenbart, wie zum Beispiel in der EP 1 342 843 A1, DE101 18 508 A1 oder der DE10 2005 062 304 A1. So wird z.B. vorgeschlagen, die Formation mit Hilfe der Entwässerungsleistung in der Formiereinheit oder durch die Retentionsmittelregelung auf einem vorgebbaren Sollniveau zu halten.

Der Erfindung liegt die Aufgabe zugrunde, ein verbessertes Verfahren sowie eine verbesserte Anlage der eingangs genannten Art zu schaffen, mit dem/der die Formation einer Faserstoffbahn auf einem höheren Niveau stabilisiert werden kann.

Die Aufgabe wird mittels des Verfahrens mit den Merkmalen des Anspruches 1 sowie einer Anlage nach Anspruch 10 gelöst.

Erfindungsgemäß wird vorgeschlagen, das Verfahren zum Regeln der Formation einer Faserstoffbahn, wie einer Papier-, Karton- oder Tissuebahn, in der Art zu verbessern, dass nicht nur die Formation der Faserstoffbahn als Regelgröße online gemessen wird und über eine automatische Formationsregelung auf einem vorgebbaren Sollniveau gehalten wird, sondern dass neben der Formation auch die Ladungskonzentration der die Faserstoffbahn bildenden Faserstoffsuspension als Regelgröße gemessen und bei der Formationsregelung berücksichtigt wird.

Die Ladungskonzentration oder Ladungsbedarf wird in der fluiden Phase der Faserstoffsuspension gemessen.

Die Formation kann dabei direkt über mindestens einen Formationssensor und/oder indirekt über wenigstens eine Hilfsgröße, insbesondere die Wassermengen in der Formiereinheit, bestimmt werden. Eine direkte Formationsmessung kann beispielsweise mit einer Kamera erfolgen.

Die Ladungskonzentration kann beispielsweise mittels einer automatisierten Polyelektrolyt-Titrations bestimmt werden.

Einen Ausgestaltung der Erfindung sieht vor, die Ladungskonzentration kontinuierlich und online zu messen, sodass jede Veränderung in der Ladungskonzentration direkt ausgeregelt werden kann. Es ist aber auch eine diskontinuierlich Messung denkbar, z.B. dann, wenn keine großen Schwankungen zu erwarten sind. Des Weiteren ist eine offline Messung denkbar.

Eine Maschine zur Herstellung einer Faserstoffbahn besteht im Wesentlichen aus einer Rohstoffaufbereitung, einer Rohstoffmischvorrichtung, einem Konstantteil, einem Blattbildungsteil, einem Blatttrocknungsteil, einem Blattveredelungsteil und einer Aufrollvorrichtung die hintereinander in Produktionsrichtung angeordnet sind. Die Messung der Ladungskonzentration erfolgt vorzugsweise an einer oder mehreren Stellen vor dem Blattbildungsteil.

In einer vorteilhaften Ausgestaltungsvariante wird die Messung der Ladungskonzentration vor dem Konstantteil bzw. hinter der Rohstoffmischvorrichtung vorgenommen. So ist der Einfluss der einzelnen Prozesschemikalien auf die Formation bestimmbar.

Des Weiteren kann die Ladungskonzentration in Produktionsrichtung gesehen vor und hinter der Zugabe von Fixiermittel erfolgt. Dabei kann besonders vorteilhaft die Wirkung des Fixiermittels auf die Suspension ermittelt und die Fixiermittelzugabe optimiert werden.

Eine weitere Möglichkeit die Formationsregelung zu verbessern ist es, die Ladungskonzentration in Produktionsrichtung gesehen zusätzlich vor und hinter der Zugabe von Retentionsmittel zu messen. So kann der Einfluss des Retentionsmittels auf die Ladungskonzentration bewertet und zur Verbesserung der Formation herangezogen und/oder die Retentionsmittelzugabe geregelt werden.

Zur Regelung der Formation und/oder der Ladungskonzentration wird erfindungsgemäß vorgeschlagen, durch entsprechendes Ändern einer oder mehrerer Stellgrößen diese auf einem vorgegebenen Sollniveau zu halten.

Da die Formation wie auch die Ladungskonzentration von sehr vielen Faktoren abhängen, können diese durch gezieltes Ändern einer oder mehrerer der folgenden Stellgrößen auf einem vorgebbaren Sollniveau gehalten werden:
- Fixiermittelzuführung
- Retentionsmittelzuführung
- Mahlungsgrad
- Zusammensetzung der Stoffmischung
- Massestärkezuführung
- Leimungsmittelzuführung (Harzleim, AKD,ASA, Polyvinylamin)
- Nassfestmittelzuführung (Melamin-Formaldehyd Kondensat, Harnstoff-Formaldehydkondensat, Epichlorhydrine etc.)
- Vakuum an der Formierwalze
- Vakuum am Formierschuh
- Stellung der Entwässerungselemente in der Siebpartie
- Lippenöffnung
- Frischwasserzugabe

Durch die zusätzliche Messung der Ladungskonzentration ist es möglich, eine der Stellgrößen gezielter zu verändern ohne den Regelkreis zur Formationsregelung nachhaltig zu destabilisieren. Dazu ist der Stellbereich der Stellgrößen vorzugsweise in einem vorgebbarem Umfang begrenzbar.

Bevorzugt wird bei Ereichen wenigstens einer vorgebbaren Grenze des Stellbereichs wenigstens einer Stellgröße Alarm ausgelöst.

Eine bevorzugte praktische Ausführungsform der erfindungsgemäßen Anlage zur Herstellung einer Faserstoffbahn, wie einer Papier-, Karton- oder Tissuebahn, umfasst einen Formationssensor, mit dem die Formation der Faserstoffbahn als Regelgröße online gemessen wird und mittels einer Formationsregelung auf ein vorgebbares Sollniveau eingestellt wird, wobei ein Ladungskonzentrationssensor zur Ladungskonzentrationsmessung vorhanden ist, mit dem die Ladungskonzentration als Regelgröße gemessen wird und bei der Formationsregelung mit einfließt.

In der Anlage wird die Formation mit einem Formationssensors z.B. einer Kamera und die Ladungskonzentration mit einem Ladungsanalysator gemessen, deren Messungen dem Formationsregler zugeführt werden. Der Formationsregler kann beispielsweise ein PID Regler sein.

Angesichts der Komplexität der Zusammenhänge bei einer Formationsregelung ist es insbesondere auch von Vorteil wenn, wenn die Anlage wenigstens einen Zustandsregler und/oder wenigstens einen Regler mit zumindest einem selbstlernenden Regelalgorithmus umfasst.

In Produktionsrichtung gesehen ist es von Vorteil, wenn in der Anlage an einer oder mehreren Stellen vor dem Former Ladungskonzentrationssensoren vorhanden sind. Bevorzugt ist ein Ladungskonzentrationssensor vor dem Konstantteil bzw. hinter der Rohstoffmischvorrichtung eingebaut.

Zur Erhöhung der Dynamik des Regelkreises ist es weiterhin von Vorteil wenn hinter und/oder vor der Rohstoffmischvorrichtung ein Ladungskonzentrationssensor vorhanden ist, sodass direkt auf Schwankungen der Ladungskonzentration eingegangen werden kann, die durch die Zugabe von Fixiermittel in die Rohstoffmischvorrichtung hervorgerufen wird.

Nachfolgend wird die Erfindung anhand von Skizzen näher erläutert. In diesen zeigen:
**Figur 1** eine schematische Darstellung einer Ausführungsform der erfindungsgemäßen Anlage zur Herstellung einer Faserstoffbahn mit einer Regelung der Formation
**Figur 2** eine schematische Darstellung des Regelungssystems einer weiteren Ausführungsform der erfindungsgemäßen Anlage zur Herstellung einer Faserstoffbahn mit einer erweiterten Regelung zur Regelung der Formation

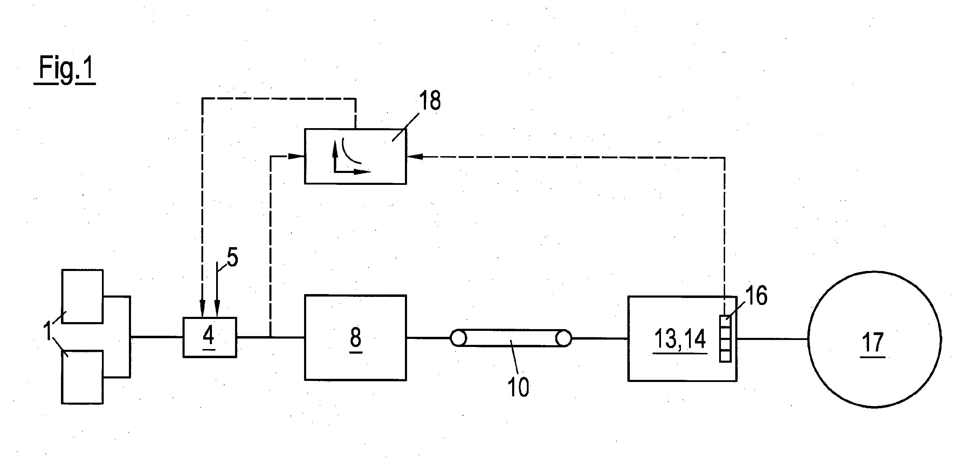

**Figur 1** zeigt in schematischer Darstellung eine beispielhafte Ausführungsform einer erfindungsgemäßen Anlage zur Herstellung einer Faserstoffbahn, bei der es sich insbesondere um eine Papier- oder Kartonbahn handeln kann.

Im vorliegenden Fall umfasst die Anlage eine Stoffaufbereitung mit einer Rohstoffmischvorrichtung 4, einen Konstantteil 8, eine Blattbildungs- und Entwässerungsvorrichtung 10, eine Blatttrocknungs- 13 und Veredelungsvorrichtung14 und einen Formationsregler 18 zur Regelung der Formation.

Über eine am Ende der Blatttrocknung 13 und Veredelung 14 vorgesehene Messeinrichtung 16 wird die Formation der Faserstoffbahn als Regelgröße online gemessen. Dies erfolgt z.B. mit einem Kamerasystem nach dem Stand der Technik. Die Ladungskonzentration in der Faserstoffsuspension wird mit einem Ladungsanalysator nach der Rohstoffmischung 4 online gemessen. Beide Werte, Formation und Ladungskonzentration, werden als Regelgrößen dem Formationsregler 18 zugeführt.

Über die automatische Formationsregelung 18 kann die Formation auf einem vorgebbaren Sollniveau gehalten werden, indem über die Formationsregelung 18 die Fixiermittelzufuhr in die Rohstoffmischvorrichtung 4 geregelt wird. Zudem können weiter Stellgrößen, wie oben aufgelistet, geändert werden um die Formation zu regeln, wobei diese nicht dargestellt sind.

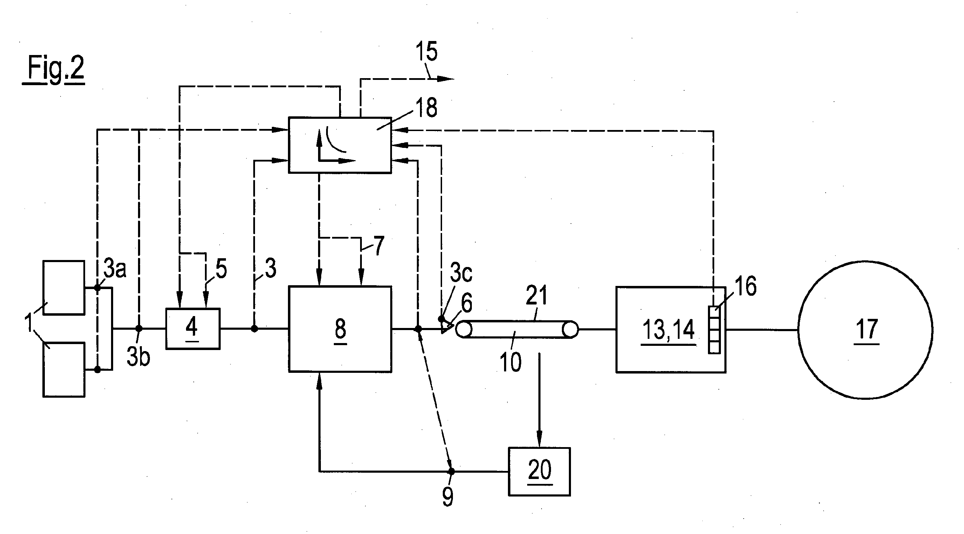

Figur 2 zeigt eine schematische Darstellung des Regelungssystems einer weiteren Ausführungsform der erfindungsgemäßen Anlage zur Herstellung einer Faserstoffbahn mit einer erweiterten Regelung zur Regelung der Formation.

Die erweiterte Regelung umfasst zusätzliche Ladungskonzentrationssensoren 3a, 3b, 3c und zudem wird die Retention 9 online gemessen und als Regelgröße der Formationsregelung zugeführt.

Die Regelung der Formation erfolgt also nicht nur aufgrund einer Messung und der Veränderung der Fixiermittelzufuhr, sondern es besteht nun die Möglichkeit aufgrund mehrer Landungskonzentrationsmessungen, an verschiednen Stellen der Suspensionsaufbereitung vor dem Former und der Retentionsmessung 9, die Formationsregelung dahingehend zu optimieren, dass die Fixiermittelzugabe wie auch die Retentionsmittelzugabe minimiert werden können.

Zur weiteren Verbesserung kann die Formationsregelung andere Stellgrößen und Einstellungen, wie oben beschrieben, verändern.

### Bezugszeichenliste

- 1: Rohstoffe
- 3, 3a, 3b ,3c: Ladungsmesspunkt
- 4: Rohstoffmischung
- 5: Fixiermittel
- 6: Stoffauflauf
- 7: Retentionsmittel
- 8: Konstantteil
- 9: Retentionsmessung
- 10: Blattbildung
- 13: Blatttrocknung
- 14: Veredlung
- 15: zu anderen Stellgrößenreglern
- 16: Formationsmessung
- 17: Aufrollung
- 18: Formationsregler
- 20: Siebwasserkreislauf
- 21: Entwässerungssensor

## Patentansprüche

1. Verfahren zum Regeln der Formation einer Faserstoffbahn, wie einer Papier-, Karton- oder Tissuebahn, bei dem die Formation der Faserstoffbahn als Regelgröße online gemessen wird und über eine automatische Formationsregelung (18) auf einem vorgebbaren Sollniveau gehalten wird
**dadurch gekennzeichnet,**
**dass** neben der Formation die Ladungskonzentration der die Faserstoffbahn bildenden Faserstoffsuspension als Regelgröße gemessen und bei der Formationsregelung (18) berücksichtigt wird und die Formation und/oder die Ladungskonzentration durch entsprechendes Ändern einer oder mehrerer Stellgrößen auf dem vorgegebenen Sollniveau gehalten werden.

2. Verfahren nach Anspruch 1
**dadurch gekennzeichnet,**
**dass** die Formation direkt über mindestens einen Formationssensor und/oder indirekt über wenigstens eine Hilfsgröße, insbesondere die Wassermengen in der Formiereinheit, bestimmt wird.

3. Verfahren nach Anspruch 1
**dadurch gekennzeichnet,**
**dass** die Ladungskonzentration mittels eines Ladungsanalysator gemessen wird.

4. Verfahren nach Anspruch 1 oder 3
**dadurch gekennzeichnet,**
**dass** die Messung der Ladungskonzentration kontinuierlich oder diskontinuierlich und online oder offline erfolgt.

5. Verfahren nach einem der vorangegangenen Ansprüche
**dadurch gekennzeichnet,**
**dass** die Messung der Ladungskonzentration in Produktionsrichtung gesehen an einer oder mehreren Stellen vor dem Blattbildungsteil (10) erfolgt.

6. Verfahren nach einem der vorangegangenen Ansprüche
**dadurch gekennzeichnet,**
**dass** die Messung der Ladungskonzentration in Produktionsrichtung gesehen vor dem Konstantteil (8) erfolgt, bzw. hinter der Rohstoffmischvorrichtung (4) erfolgt.

7. Verfahren nach Anspruch 6
**dadurch gekennzeichnet,**
**dass** die Messung der Ladungskonzentration in Produktionsrichtung gesehen vor und hinter der Zugabe von Fixiermittel (5) erfolgt.

8. Verfahren nach einem der vorangegangenen Ansprüche
**dadurch gekennzeichnet,**
**dass** die Messung der Ladungskonzentration in Produktionsrichtung gesehen vor und hinter der Zugabe von Retentionsmittel (7) erfolgt

9. Verfahren nach Anspruch 1
**dadurch gekennzeichnet,**
**dass** zumindest eine der folgenden Stellgrößen entsprechend geändert wird:
- Fixiermittelzuführung (5)
- Retentionsmittelzuführung (7)
- Mahlungsgrad
- Zusammensetzung der Stoffmischung
- Massestärkezuführung
- Leimungsmittelzuführung (Harzleim, AKD,ASA, Polyvinylamin)
- Nassfestmittelzuführung (Melamin-Formaldehyd Kondensat, Harnstoff-Formaldehydkondensat, Epichlorhydrine)
- Vakuum an der Formierwalze
- Vakuum am Formierschuh
- Stellung der Entwässerungselemente in der Siebpartie
- Lippenöffnung (6).

10. Anlage zur Herstellung einer Faserstoffbahn, wie einer Papier-, Karton- oder Tissuebahn, mit einer Formationsmessung, bei der die Formation der Faserstoffbahn als Regelgröße online gemessen wird und über eine automatische Formationsregelung auf ein vorgebbares Sollniveau einstellbar ist, zur Durchführung des Verfahrens nach einem der vorhergehenden Ansprüche,
**dadurch gekennzeichnet,**
**dass** ein Ladungskonzentrationssensor (3, 3a, 3b, 3c) zur Ladungskonzentrationsmessung vorhanden ist, mit dem die Ladungskonzentration der die Faserstoffbahn bildenden Faserstoffsuspension als Regelgröße neben der Formation gemessen und bei der Formationsregelung (18) berücksichtigt wird.

11. Anlage nach Anspruch 10
**dadurch gekennzeichnet,**
**dass** die Formation mit einem Formationssensors und die Ladungskonzentration mit einem SCD-Sensor (3, 3a, 3b, 3c) gemessen wird.

12. Anlage nach Anspruch 10
**dadurch gekennzeichnet,**
**dass** in Produktionsrichtung gesehen an einer oder mehreren Stellen vor dem Former (10) Ladungskonzentrationssensoren (3, 3a, 3b, 3c) vorhanden sind.

13. Anlage nach Anspruch 12
**dadurch gekennzeichnet,**
**dass** in Produktionsrichtung gesehen vor dem Konstantteil (8) bzw. hinter der Rohstoffmischvorrichtung (4) ein Ladungskonzentrationssensor (3, 3a, 3b, 3c) vorhanden ist.

14. Anlage nach Anspruch 12
**dadurch gekennzeichnet,**
**dass** in Produktionsrichtung gesehen hinter und/oder vor der Rohstoffmischvorrichtung (4) ein Ladungskonzentrationssensor (3, 3a, 3b, 3c) vorhanden ist.

## Claims

1. Method for regulating the formation of a fibrous web, such as a paper, board or tissue web, in which the formation of the fibrous web is measured online as controlled variable and is kept at a predefinable intended level via an automatic formation control system (18),
**characterized in that**
in addition to the formation, the charge concentration of the fibrous suspension forming the fibrous web is measured as controlled variable and taken into account in the formation control system (18), and the formation and/or the charge concentration are kept at the predefined intended level by means of appropriately changing one or more manipulated variables.

2. Method according to Claim 1,
**characterized in that**
the formation is determined directly via at least one formation sensor and/or indirectly via at least one auxiliary variable, in particular the quantities of water in the forming unit.

3. Method according to Claim 1,
**characterized in that**
the charge concentration is measured by means of a charge analyser.

4. Method according to Claim 1 or 3,
**characterized in that**
the measurement of the charge concentration is carried out continuously or discontinuously and online or off-line.

5. Method according to one of the preceding claims,
**characterized in that**
as viewed in the production direction, the measurement of the charge concentration is carried out at one or more points before the sheet forming part (10).

6. Method according to one of the preceding claims,
**characterized in that**
as viewed in the production direction, the measurement of the charge concentration is carried out before the approach system (8) and after the raw material mixing device (4).

7. Method according to Claim 6,
**characterized in that**
as viewed in the production direction, the measurement of the charge concentration is carried out before and after the addition of fixing agent (5).

8. Method according to one of the preceding claims,
**characterized in that**
as viewed in the production direction, the measurement of the charge concentration is carried out before and after the addition of retention aid (7).

9. Method according to Claim 1,
**characterized in that**
at least one of the following manipulated variables is changed appropriately:
- fixing agent supply (5)
- retention aid supply (7)
- level of refining
- composition of the stock mixture
- supply of body starch
- sizing agent supply (rosin size, AKD, ASA, polyvinyl amine)
- wet strength agent supply (melamine formaldehyde condensate, urea formaldehyde condensate, epichlorhydrin)
- vacuum on the forming roll
- vacuum on the forming shoe
- position of the dewatering elements in the wire section
- lip opening (6).

10. Plant for producing a fibrous web, such as a paper, board or tissue web, having a formation measurement, in which the formation of the fibrous web is measured online as controlled variable and can be adjusted to a predefinable intended level via an automatic formation control system, for carrying out the method according to one of the preceding claims,
**characterized in that**
there is a charge concentration sensor (3, 3a, 3b, 3c) for charge concentration measurement, with which the charge concentration of the fibrous suspension forming the fibrous web is measured as controlled variable in addition to the formation and is taken into account in the formation control system (18).

11. Plant according to Claim 10,
**characterized in that**
the formation is measured with a formation sensor and the charge concentration with an SCD sensor (3, 3a, 3b, 3c).

12. Plant according to Claim 10,
**characterized in that**
as viewed in the production direction, there are charge concentration sensors (3, 3a, 3b, 3c) at one or more points before the former (10).

13. Plant according to Claim 12,
**characterized in that**
as viewed in the production direction, there is a charge concentration sensor (3, 3a, 3b, 3c) before the approach system (8) and after the raw material mixing device (4).

14. Plant according to Claim 12,
**characterized in that**
as viewed in the production direction, there is a charge concentration sensor (3, 3a, 3b, 3c) after and/or before the raw material mixing device (4).

## Revendications

1. Procédé de réglage de la formation d'une nappe fibreuse, telle qu'une nappe de papier, de carton ou de papier tissu, dans lequel la formation de la nappe fibreuse est mesurée en ligne en tant que grandeur de réglage et maintenue par le biais d'un réglage de formation automatique (18) à un niveau de consigne prédéfinissable,
**caractérisé en ce**
**qu'**outre la formation, la concentration de charge de la suspension fibreuse formant la nappe fibreuse est mesurée en tant que grandeur de réglage et est prise en compte lors du réglage de formation (18) et la formation et/ou la concentration de charge sont maintenues au niveau de consigne prédéfini par variation correspondante d'une ou de plusieurs grandeurs de commande.

2. Procédé selon la revendication 1,
**caractérisé en ce que**
la formation est déterminée directement par le biais d'au moins un capteur de formation et/ou indirectement par le biais d'au moins une grandeur auxiliaire, en particulier la quantité d'eau dans l'unité de formage.

3. Procédé selon la revendication 1,
**caractérisé en ce que**
la concentration de charge est mesurée au moyen d'un analyseur de charge.

4. Procédé selon la revendication 1 ou 3,
**caractérisé en ce que**
la mesure de la concentration de charge s'effectue de manière continue ou discontinue et en ligne ou hors ligne.

5. Procédé selon l'une quelconque des revendications précédentes,
**caractérisé en ce que**
la mesure de la concentration de charge s'effectue, vu dans la direction de production, au niveau d'un ou de plusieurs endroits avant la partie de formation de feuilles (10).

6. Procédé selon l'une quelconque des revendications précédentes,
**caractérisé en ce que**
la mesure de la concentration de charge s'effectue, vu dans la direction de production, avant la partie constante (8) ou derrière le dispositif de mélange de matières premières (4).

7. Procédé selon la revendication 6,
**caractérisé en ce que**
la mesure de la concentration de charge s'effectue, vu dans la direction de production, avant et après l'ajout d'agents de fixation (5).

8. Procédé selon l'une quelconque des revendications précédentes,
**caractérisé en ce que**
la mesure de la concentration de charge s'effectue, vu dans la direction de production, avant et après l'ajout d'agents de rétention (7).

9. Procédé selon la revendication 1,
**caractérisé en ce**
**qu'**au moins l'une des grandeurs de commande suivantes est modifiée de manière correspondante :
- alimentation en agents de fixation (5),
- alimentation en agents de rétention (7),
- degré de broyage,
- composition du mélange de matières,
- alimentation en amidon,
- alimentation en agents d'encollage (colle de résine, AKD, ASA, polyvinylamine),
- alimentation en agents de résistance à l'humidité (condensat de mélamine-formaldéhyde, condensat d'urée-formaldéhyde, épichlorhydrine),
- vide au niveau du rouleau de formage,
- vide au niveau du sabot de formage,
- position des éléments d'égouttage dans la section de toile,
- ouverture de lèvre (6).

10. Installation de fabrication d'une nappe fibreuse, telle qu'une nappe de papier, de carton ou de papier tissu, comprenant une mesure de formation, dans laquelle la formation de la nappe fibreuse est mesurée en ligne en tant que grandeur de réglage et peut être ajustée par le biais d'un réglage de formation automatique à un niveau de consigne prédéfinissable pour mettre en oeuvre le procédé selon l'une quelconque des revendications précédentes,
**caractérisée en ce**
**qu'**un capteur de concentration de charge (3, 3a, 3b, 3c) est prévu pour la mesure de la concentration de charge, avec lequel la concentration de charge de la suspension de matière fibreuse formant la nappe fibreuse est mesurée en tant que grandeur de réglage en plus de la formation et est prise en compte lors du réglage de formation (18).

11. Installation selon la revendication 10,
**caractérisée en ce que**
la formation est mesurée avec un capteur de formation et la concentration de charge est mesurée avec un capteur SCD (3, 3a, 3b, 3c).

12. Installation selon la revendication 10,
**caractérisée en ce que**
vu dans la direction de production, au niveau d'un ou de plusieurs endroits avant le formeur (10), sont prévus des capteurs de concentration de charge (3, 3a, 3b, 3c).

13. Installation selon la revendication 12,
**caractérisée en ce que**
vu dans la direction de production, avant la partie constante (8) ou derrière le dispositif de mélange de matières premières (4), est prévu un capteur de concentration de charge (3, 3a, 3b, 3c) .

14. Installation selon la revendication 12,
**caractérisée en ce que**
vu dans la direction de production, derrière et/ou avant le dispositif de mélange de matières premières (4), est prévu un capteur de concentration de charge (3, 3a, 3b, 3c).
